# EUROPEAN PATENT APPLICATION

(11) **EP 1 063 286 A1**
(43) Date of publication of application: **27.12.2000**
(21) Application number: 99901955.7
(22) Date of filing: 08.02.1999
(51) Int. Cl.: C12M 1/00, C12Q 1/68, C12N 15/00

(54) **SUBSTRATES FOR IMMOBILIZING AND AMPLIFYING DNA, DNA-IMMOBILIZED CHIPS HAVING DNA IMMOBILIZED ON THE SUBSTRATES, AND METHOD FOR AMPLIFYING DNA**

(30) Priority: 09.02.1998 JP 4103598
(71) Applicant: Toyo Kohan Co., Ltd, Tokyo 100-8911 (JP)
(72) Inventor: TANGA, Michifumi, Toyo Kohan Co., Ltd., Kudamatsu-shi, Yamaguchi-ken 744-8611 (JP); TAKAHASHI, Kojiro, Hiroshima-shi, Hiroshima-ken 734-0015 (JP)
(74) Representative: Jaenichen, Hans-Rainer, Dr.
(86) International application number: PCT/JP99/00524
(87) International publication number: WO 99/40173

(57) **Abstract**

A purpose of the present invention is to provide substrates utilized for a library of DNA by immobilizing DNA and DNA-immobilized chips utilized for reproducing DNA by a PCR amplifying reaction. In the present invention, PCR amplifying method is operated by utilizing DNA-immobilized chips having DNA immobilized on an excellent conductive substrate. A surface of the substrate is chemically modified with a radical of which a terminal is a polar radical. In the PCR method, DNA can be amplified for a short period by utilizing DNA-immobilizing chips having DNA immobilized on the substrates.

## Description

### FIELD OF THE INVENTION

The present invention relates to a substrate for immobilizing DNA, peptide and so on utilized in a molecular biology field and a biochemical field, particularly to substrates with excellent heat conductivity, more particularly to substrates chemically modified with hydroxyl group, carboxyl group, epoxy group, amino group and so on (CHO) at a terminal end of the substrates and DNA-immobilized chips having DNA immobilized on the substrates.

### BACKGROUND OF THE INVENTION

In prior art, in order to obtain a specific amount of DNA, a heat cycle including following steps 1) to 3) has to be repeated in a DNA amplifying step.
1) A temperature of a test piece is increased to 95°C for untying hydrogen bond of double DNA chains;
2) The temperature of the test piece is decreased to 45°C for reunifying to a primer in order to reproduction DNA; and
3) The temperature of the test piece is increased to 74°C so as to reproduce DNA by extending primer by extending heat-resistant polymerize.

In such a DNA amplifying reaction by a method so called as PCR, a test piece with reaction solution is contained in a tube-shaped plastic reaction container. The container is stocked in an aluminium block and the above heat cycles are repeated.

However, the above amplifying reaction is repeatedly heated and cooled DNA with reaction solution so that long time is needed so as to obtain a specific amount of DNA. In addition, thermal accuracy for controlling the reaction solution is low so that another DNA except target DNA is also reproduced.

To resolve the above drawbacks, a subject of the present invention is to provide the most preferable solid-state substrate for easily immobilizing DNA and reproducing DNA in accordance with the DNA amplifying reaction, DNA-immobilized chips having DNA immobilized on the substrates, and a method for amplifying DNA.

### DISCLOSURE OF THE INVENTION

Substrates according to the present invention is characterized of having excellent thermal conductivity for immobilizing and amplifying DNA. These substrates are preferably diamond and diamond chemically modified.

These substrates are preferably chemical modified with polar group, carboxyl group, epoxy group or amino group (CHO) at a terminal end of the substrates.

It is preferable that the carboxyl group is connected to a surface of the substrate through an ester bond or a peptide (amide) bond, a carboxyl group is connected to a surface of the substrate with silane coupling agent or an epoxy group or an amino group is connected to a surface of the substrate with silane coupling agent.

DNA-immobilized chips according to the present invention are characterized of having DNA immobilized on a surface of the substrates.

A method for amplifying DNA according to the present invention is characterized of amplifying DNA with these substrates or chips.

### BEST MODE FOR CARRYING OUT THE INVENTION

A substrate according to the present invention is a solid-state substrate for immobilizing and amplifying DNA and preferably has excellent thermal conductivity. For example, diamond is material having one of the best thermal conductivity of the all materials so that diamond can be heated up/cooled down rapidly. By providing the substrates according to the present invention, a heat cycle period for repeatedly heating/cooling such as DNA amplifying reaction can be shortened.

In substrates according to the present invention, hydroxyl group, carboxyl group, epoxy group, amino group and so on (CHO) are chemically modified at a surface of the substrate.

DNA and so on are immobilized easily so that the substrates according to the present invention are the most suitable material so as to replicate DNA by DNA amplifying reaction.

In the case that a surface of the substrate is contaminated, chips according to the present invention can replicate chemical modification by hydrolyzing.

In the substrates according to the present invention, a thermal conductivity ratio of a solid-state substrate is preferably equal to or more than 0.1W/cm·K.

More preferably, the thermal conductivity ratio is equal to or more than 0.5W/cm·K. Further, more preferably, the thermal conductivity ratio is equal or more than 1w/cm· K. If the thermal conductivity ratio of the substrate is equal to or more than 0.1W/cm·K, heating and cooling can be changed smoothly in the case that the DNA-immobilized substrates according to the present invention are reacted by PCR (polymerize-chain-reaction).

As an example of good thermal conductivity material, diamond, metals such as silver, copper, aluminum, tungsten, molybdenum and so on can be considered. Ceramic such as alumina, aluminum nitride, titanium carbide, silicon carbide, silicone and so on can be also considered.

Materials mixed with the above described material and ceramic can be also suitable. Further, plastic material such as polycarbonate and fluorine resin can be suitable.

If material is chemically suitable, the other materials may be suitable in addition to the above described metal, ceramic and plastic. For example, diamond and diamond-like can be suitable. Material mixed with plastic and the above described metal, ceramic and diamond can be suitable.

As a material of a substrate made of diamond, synthetic diamond, high pressurized synthetic diamond or natural diamond can be utilized. These kinds of diamond may have monocrystal substance or polycrystal substance. In view of productivity, diamond produced by a vapor phase composite method such as microwave plasma CVD method is preferable.

A method for forming a substrate according to the present invention may be selectable. For example, a microwave plasma CVD method, an ECRCVD method, a high frequency plasma CVD method, an IPC method, a DC spattering method, an ECR spattering method, an ion plating method, an ark ion plating method, an EB deposition method, a heat resistant deposition method are considered. It is also considered a method for mixing metal powder, ceramic powder or other material with binder such as resin. It is also considered a method for pressurizing metal powder, ceramic powder or other material by a press forming machine and sintering pressurized material at high temperature.

It is preferable that a surface of a substrate according to the present invention is consciously roughed. Such a surface is suitable for immobilizing large amount of DNA since an area of the roughed surface is enlarged compared to a smooth surface. A shape of the substrate is not restricted. A plate shape, a thread shape, a ball shape, a polygon shape, powder shape and so on can be considered.

Further, a composite type with these substrates and the other material such as a double layered type can be also considered. In the substrates according to the present invention, a surface of the substrate is chemically modified. It is preferable that a polar group, hydroxyl group or carboxyl group is comprised at a terminal end of the chemical modification. That is, a specific group is added (chemically modified) on a surface of the substrate. By providing such chemical modification, DNA becomes to be easily immobilized on a surface of the substrate. As a specific group chemically modified on the surface of the substrate and having the polar group at the terminal end, hydroxyl group, carboxyl group, sulfuric group, cyano group, nitro group, thiol group, amino group, epoxy group and so on can be considered. In addition, organic carbonic acid can be considered. In the above described groups, carboxyl group may be directly added to a substrate such as diamond or indirectly added to a substrate through the other hydroxyl group at a terminal end.

In such a case, hydroxyl group having from 1 to 10 carbons is preferable in order to immobilize DNA. As acid for changing to hydrocarbon radical, monocarbonic acid such as formic acid, acetic acid, and propionic acid, dicarbonic acid such as oxalic acid, malonic acid, succinic acid, maleic acid, fumaric acid, phthalic acid, and polyatomic carbonic acid such as trimellitic acid can be considered.

In the case that a substrate according to the present invention is utilized for DNA amplifying reaction by PCR method, there are two cases. One is a first case in which anti-hydrolysis characteristic is required and another is a second case in which chemical modification is reproduced by hydrolysis.

In the case of requiring anti-hydrolysis characteristic, it is preferable that a radical in which carboxyl radical is connected to a terminal end of the above hydrolysis radical is connected to a surface of a substrate through a peptide (amide) linkage so as to provide anti-alkaline characteristic.

On the other hand, in the case of removing produced chemical modification by hydrolysis and reproducing, it is preferable that a radical in which carboxyl radical is connected to a terminal end of the above hydrolysis radical is connected to a surface of a substrate through an ester linkage so as to provide hydrolysis characteristic in alkaline solution.

As a method for linking a hydroxyl radical connected to a terminal end of the hydrocarbon radical to a surface of a substrate, it can be considered a method for oxidizing a surface of a substrate with oxygen plasma and then steaming, a method for chloridizing a surface of a substrate by irradiating ultraviolet light in chlorine gas and then hydroxylating in alkaline solution and a method for oxidizing a surface of a substrate with oxygen plasma, chloridizing and then hydroxylating in alkaline solution.

In the case of linking hydroxyl radical to a surface of a substrate, chemical modification with carboxyl radical and so on becomes more strength by treating with silane coupling agent, titanium coupling agent and aluminum coupling agent.

As a method for linking a radical in which carboxyl radical is connected to a terminal end of a hydrocarbon radical is connected to a surface of a substrate through a peptide (amide) linkage, it can be considered a method for chloridizing a surface of a substrate by irradiating ultraviolet radiation in chlorine gas, aminating the radical by irradiating ultraviolet radiation in anmonia gas, reacting with carbonic chloride in nonaqueous solvent and then neutralizing the radical in alkalescent solution.

As a method for linking a radical in which carboxyl radical is connected to a terminal end of a hydrocarbon radical is connected to a surface of a substrate through an ester linkage, it can be considered that a method for chloridizing a surface of the substrate by irradiating ultraviolet radiation in chlorine gas, reacting with carboxylic soda in nonaqueous solvent and then neutralizing the substrate weak acid solvent or a method for oxidizing a surface of the substrate with oxygen plasma, chloridizing, hydrolyzing in alkali solution, reacting with carbonic chloride in nonaqeous solvent and then neutralizing the substrate in alkalescent solution. Embodiments of the present invention will be described in detail hereinafter.

### (EXAMPLE 1)

A vapor phase synthetic diamond of which diameter is 64mm and a thickness is 0.3mm is produced by a microwave plasma CVD method. Then, the diamond is polished so as to have a uniform thickness of 0.25mm. With respect to a polished surface of 10mm x 10mm, absorption strength of 2879cm⁻¹ which is resulted from stretching vibration of carbon and hydrogen is measured by FTIR method (Fourier transform infrared spectroscopic analysis method), eachmeasured value is almost constant. Several pieces in size of 10mm x 10mm are cut from the diamond by laser beam. Each piece is utilized as a test piece of embodiments 1 to 6. In the embodiment 1, a surface of the diamond is oxidized with oxygen plasma energized by microwave. Then, the test piece is set in a separable flask and air in the separable flask is substituted with steam. While steam is filled into the separable flask, the flask is heated to 400°C for 30miniutes and then cooled down.

After the test piece in the separable flask is picked up and dried in order to obtain diamond having hydroxyl radical at a terminal end. After polishing a surface of the diamond, the diamond is treated with oxygen plasma and steam, peak strength of hydroxyl radical is measured by a SIMS method (secondary ion mass analysis method). Upon comparing with a peak strength of hydrogen as 1, the peak strength of the hydroxyl radical is shown in Table 1.

**Table 1**

| Process | Peak strength of hydroxyl radical |
|---|---|
| After polishing a surface | 0.14 |
| After Oxygen plasma treatment | 0.70 |
| After steam treatment | 1.14 |

As shown in Table 1, the peak strength of the hydroxyl radical is increased by the oxygen plasma treatment and the steam treatment. As the result, it is recognized that a surface of diamond is chemically modified with hydroxyl radical.

### (EXAMPLE 2)

After polishing a surface of vapor phase synthetic diamond obtained in the embodiment 1, a test piece in size of 10mm x 10mm is cut off by laser beam. The test piece is set in a separable flask and air in the separable flask is substituted with argon gas. While chloride gas of which flow rate is 1 SCCM, the surface of the diamond is chloridized by ultraviolet beam irradiated Hg-Xe lamp of which main wavelength is 3600 Å for 60minutes. After substituting the air in the flask with argon gas, the test piece is picked up. After boiling the test piece in sodium hydroxide solution of 10wt% for 15minutes, the test piece is cleaned and dried in order to obtain a diamond having hydroxyl radical at a terminal end. After polishing the surface of the diamond and chloridizing the diamond by the SIMS method, peak strengths of hydrogen, hydroxyl radical and chlorine radical is measured, respectively. In the case that the peak strength of hydrogen is 1, each measured peak strength of hydroxyl radical and chlorine radical are shown in Table 2.

**Table 2**

| Process | Ratio of peak strength | |
|---|---|---|
| | Hydroxyl radical | Chlorine radical |
| After polishing a surface | 0.14 | - |
| After chlorodizing | 0.20 | 0.50 |
| After sodium hydroxide treatment | 0.47 | 0.35 |

As shown in Table 2, the peak sterngth of the hydroxyl radical is increased by chrolodizing and treating with sodium hydroxide. As the result, it is recognized that a surface of diamond is chemically modified with hydroxyl radical.

Judging from the reduction of chlorine radical, it can be recognized that the chlorine radical is substitute by hydroxyl radical.

### (EXAMPLE 3)

A surface of a vapor phase synthetic diamond obtained in the embodiment 1 is polished. A test piece in size of 10mm x 10mm is cut off by laser beam. After oxidizing a surface of the diamond with oxygen plasma energized by micro wave, the surface of the diamond is chloridized as similar as the diamond in the embodiment 2. After substituting atmosphere in a flask with argon gas, the test piece is picked up. The test piece is boiled in sodium hydroxide solution (10wt%) for 15 minutes, cleaned and then dried. As the result, a diamond with hydroxyl radical at a terminal end can be obtained. In accordance with the SIMS method, a surface of a diamond is polished, treated with oxygen plasma, chlorodized and treated with sodium hydroxide, peak strength of hydrogen, hydroxyl radical and chloride radial is measured, respectively. In the case that the peak strength of hydrogen is 1, each measured peak strength of hydroxyl radical and chloride radical is shown in Table 3, respectively.

**Table 3**

| Process | Ratio of peak strength | |
|---|---|---|
| | Hydroxyl radical | Chloride radical |
| After polishing surface | 0.14 | - |
| After oxygen plasma treatment | 0.70 | - |
| After chlorodizing | 0.21 | 0.47 |
| After sodium hydroxide treatment | 0.54 | 0.33 |

As shown in Table 3, the peak strength of hydroxyl radical is increased by the oxygen plasma treatment, chlrodization, and sodium hydroxide treatment. AS the result, a surface of the diamond is chemically modified with hydroxyl radical.

Judging from the reduction of chloride radical, it is recognized that the chloride radical s substituted by hydroxyl radical.

### (EXAMPLE 4)

After polishing a surface of vapor phase synthetic diamond obtained in the embodiment 1, a test piece in size of 10mm x 10mm is cut off by laser beam. The test piece is set in a separable flask and air in the separable flask is substituted with argon gas. While chloride gas of which flow rate is 1SCCM, the surface of the diamond is chloridized by ultraviolet beam irradiated Hg-Xe lamp of which main wavelength is 3600 Å for 60minutes. After substituting the air in the flask with argon gas again, N,N-dimethyl formaldehyde solution of sebacic soda of 1wt% of 100ml is added. A condenser is set in the separable flask so as to reflux for 2 hours. After picking up the test piece, the test piece is cleaned in acetic acid solution (1wt%), cleaned with acetone and then dried. As the result, a diamond with carboxyl radical connected to a terminal end in which sebacic acid is connected to ester linkage. In accordance with the SIMS method, a surface of a diamond is polished, chloradized and treated with sebacic soda, peak strengths of hydrogen, hydroxyl radical and chlorine radical is measured, respectively. In the case that the peak strength of hydrogen is 1, each measured peak strength of hydroxyl radical and chlorine radical is shown in Table 4.

**Table 4**

| Process | Ratio of peak strength | |
|---|---|---|
| | Hydroxyl radical | Chlorine radical |
| After polishing a surface | 0.14 | - |
| After chloridizing | 0.20 | 0.50 |
| After sebacic soda treatment | 0.40 | 0.34 |

As shown in Table 4, the peak strength of the hydroxyl radical is increased by chloridizing and the sebacic soda treatment.

In accordance with a FTIR method, absorption strength which is resulted from stretching vibration of carbon and hydrogen and absorption strength which is resulted from stretching vibration of carbon and oxygen are measured by FTIR method.

As the result each absorption strength is increased (ratio of absorption strength is increased to 30% with respect to diamond blank is about 30%).

Thereby, it can be recognized that a surface of a diamond is chemically modified with a radical in which carboxyl radical is connected at a terminal end of hydrocarbon radical of sebacic acid.

It is recognized that the chlorine radical is substituted by the hydroxyl radical since the number of chlorine radical was decreased.

### (EXAMPLE 5)

A surface of a vapor phase synthetic diamond obtained in the embodiment 1 is polished. A test piece in size of 10mm x 10mm is cut off by laser beam. After oxidizing a surface of the diamond with oxygen plasma energized by micro wave, the surface of the diamond is chloridized as similar as the diamond in the embodiment 2. After substituting atmosphere in a flask with argon gas, the test piece is picked up. The test piece is boiled in patassium hydroxide solution (10wt%) for 15minutes so as to modify hydroxyl radical at a surface of a diamond. After drying, a test piece is set in a separable flask in which a condenser with a calcium chloride dry pipe at an upper portion thereof is arranged. By adding chloroform of 50ml and triethylamine of 1g, atmosphere in the flask is substituted with argon gas. While the separable flask is cooled with ice, solution of chloroform 50ml and succinyl chloride of 10g is gradually added. After refluxing for 4 hours, the test piece is picked up, cleaned with potassium carbonate solution of 10wt%, cleaned with acetone and then dried. As the result, it is obtained a diamond with malonic acid connected through ester linkage of which a terminal end is connected to a carboxyl radical.

In accordance with the SIMS method, a surface of a diamond is polished, treated with oxygen plasma, chloridized, hydroxylated and treated with succinyl chloride, peak strength of hydrogen, hydroxyl radical is measured, respectively. In the case that the peak strength of hydrogen is 1, a measured peak strength of hydroxyl radical is shown in Table 5.

**Table 5**

| Process | Peak strength ratio of hydroxyl radical |
|---|---|
| After polishing a surface | 0.14 |
| After oxygen plasma treatment | 0.70 |
| After chlorodizing | 0.21 |
| After hydroxylating | 0.70 |
| Succinyl chloride treatment | 0.50 |

As shown in Table 5, the peak strength of the hydroxyl radical is increased by oxygen plasma treatment, chloridizing, hydroxylating and the succinyl chloride treatment. In accordance with a FTIR method, absorption strength which is resulted from stretching vibration of carbon and hydrogen and absorption strength which is resulted from stretching vibration of carbon and oxygen are measured. As the result each absorption strength is increased (increasing ratio of absorption strength with respect to diamond blank is about 25%).

Thereby, it can be recognized that a surface of a diamond is chemically modified with a radical in which carboxyl radical is connected at a terminal end of hydrocarbon radical of malonic acid.

### (EXAMPLE 6)

A surface of a vapor phase synthetic diamond obtained in the embodiment 1 is polished. A test piece in size of 10mm x 10mm is cut off by laser beam. After oxidizing a surface of the diamond with oxygen plasma energized by micro wave, the surface of the diamond is chloridized as similar as the diamond in the embodiment 2.

Atmosphere in a separable flask in which the test piece is set is substituted with argon gas. While ammonia gas of which flow rate is 1SCCM is filled into the flask, the surface of the diamond is modified with amino acid by irradiating ultraviolet beam produced by a Hg-Xe lamp of which main wavelength is 3600 Å for 60minutes. After substituting the air in the flask with argon gas, a condenser with a calcium chloride dry pipe is provided at an upper portion of the separable flask. Chloroform of 50ml is added into the flask and then atmosphere in the flask is substituted with argon gas.

In the next, while the separable flask is cooled among ice blocks, chloroform solution of 50ml with succinyl chloride of 10g is gradually added. After refluxing for 4hours, the test piece is picked up. After cleaning the test piece with potassium carbonate solution (10wt%), the test piece is cleaned with acetone and dried. As the result, a diamond connected with malonic acid through peptide linkage of which a terminal end is connected to a carboxyl radical. In accordance with SIMS method, peak strengths of hydrogen, hydroxyl radical arid chlorine radical is measured before each treatment, respectively. In the case that the peak strength of hydrogen is 1, each measured peak strength of hydroxyl radical and chlorine radical are shown in Table 6.

**Table 6**

| Process | Ratio of peak strength | |
|---|---|---|
| | Hydroxyl radical | Chloride radical |
| After hydrogen plasma treatment | 0.06 | - |
| After chloridizing | 0.21 | 0.47 |
| After amino toreatment | 0.18 | 0.10 |
| After succinyl chloride treatment | 0.58 | 0.10 |

As shown in Table 6, the peak strength of the hydroxyl radical is increased by hydrogen plasma treatment, chloridizing, hydroxylating and the succinyl chloride treatment. In accordance with a FTIR method, absorption strength which is resulted from stretching vibration of carbon and hydrogen and absorption strength which is resulted from stretching vibration of carbon and oxygen are measured. As the result, each absorption strength is increased (increasing ratio of absorption strength with respect to diamond blank is about 25%). Thereby, it can be recognized that a surface of a diamond is chemically modified with a radical in which carboxyl radical is connected at a terminal end of hydrocarbon radical of malonic acid.

Regarding diamonds with a radical of which a terminal end is chemically modified with carboxyl radical obtained by the embodiments 1 through 6, reaction for amplifying DNA is treated with the diamonds. After passing one hour, a predetermined amount of DNA can be obtained.

Regarding the diamond chip chemically modified according to the present invention, a terminal chloride radical of oligo nucleic acid may be immobilized at a terminal hydroxyl radical or a terminal carboxyl radical with hydrogen bond, then DNA of which chloride arrangement is relatively supplement with respect to the oligo nucleic acid may be immobilized so as to utilize as a DNA library chip.

Alternatively, it may utilize as a library by immobilizing nucleotide, oligonucleotide, DNA flagment and so on a diamond surface.

### (EXAMPLE 7)

Titanium carbide circular board of which a diameter is 64mm, a thickness is 0.1mm and surface roughness is RA=0.1mm, is vapor phase synthesized by a high frequency plasma CVD method.

A test piece of 3mmx5mm is cut from the circular board by laser beam. A result of measuring thermal conductivity ratio is 0.44W/cm·K.

In the embodiment 7, a surface of the test piece is oxidized with oxygen plasma energized by high frequency. Then, the test piece is set in a separable flask. After substituting air in the flask by steam, the flask is heated up to 400°C for 30 min while steam is flown in the flask, the flask is left as it is for cooling. The test piece is picked up and dried. As the result, a substrate of which a terminal end having a hydroxyl radical can be obtained. Further, a surface of the substrate is dipped in a silane coupling solution so as to obtain a substrate covered with silane coupling agent. The substrate is directly contacted with a Peltier element as heating/cooling means so as to thermally control the substrate. Then, the following PCR method is operated.

### (DNA immobilization)

In the case of mRNA (messenger RNA), oligo-dT₁₆₋₂₀ is immobilized on a surface of the substrate. On the other hand, in the case of gDNA (genomic DNA), olegonucleotide having target limit enzyme portion is immobilized by chemical ester linkage reaction. In the next, in the case of immobilizing cDNA (complementary DNA), mRNA and immobilized oligo-dT₁₆₋₂₀ is hybridized with all RNA solution extracted from tissue and cell at a low temperature from 0°C to 4°C. Then, the substrate is thermally controlled from 37°C to 60°C and cDNA synthesized by RT enzyme (Reverse Transcriptase). In such a case, cDNA immobilized by extending toward 5' of immobilized oligo-dT₁₆₋₂₀ is utilized.

Solution of synthdized immobilized cDNA ad mRNA in hybrid condition is heated to 90°C so as to dehybridize mRNA, reaction solution is substituted by TE buffer solution (Tris-EDTA), the substrate is again colled at a low temperature from 0°C to 4°C and cleaned with ethanol. As the result, refined immobilized cDNA chip in a condition of a single chain is produced.

In the case of gDNA immobilization, as similar as the above oligo-dT₁₆₋₂₀, immobilized olegonucleotide having a target limit enxyme portion is immobilized on a surface of the substrate according to the present invention. In the next, reaction solution for chemically immobilization is substituted by reaction solution including hybrid side olegonucleotide and a target limit enzyme at a low temperature from 0°C to 4°C. After hybridization between each olegonucleotide, the substrate is heated to 37°C so as to cut the semi-solidified limit enzyme of olegonucleotide.

After cutting the limit enzyme, a temperature of the substrate is shifted to a range of from 0°C to 4°C. The solution is substituted by reaction solution including a cut piece of gDNA with the target limit enzyme and Ligase enzyme. The temperature of the substrate is again increased to 37°C so as to act Ligase reaction. Thus, gDNA immobilized chip in a condition of a single chain is produced.

Regarding cDNA immobilized chips above produced on a surface of the substrate or gDNA immobilized chips above produced on a surface of the substrate, these chips can be separated to different containers corresponding to its respective purpose described below. purpose described below.
① Comparison of gene variation of the same kind of tissue and cell for a plurality of test pieces;
② Comparison of occurrence and change of gene of each tissue and cell of the same test piece; and
③ Comparison of occurrence and change of gene in the same test piece corresponding to spending time after medical care and sergeant.

For example, in the case of ①, in order to compare gene variation of the same kind of tissue and cell, a plurality of test piece (a plurality of DNA immobilized chips) are set in the different containers, respectively. These plurality of connected containers is as one cassette.

Theses cassettes are embedded at a body of a reactor. If at least two cassettes are systematically produced. The comparison of the test pieces can be compared effectively.

The above one cassette in which a plurality of container are connected or the above plurality of cassettes is designated one aggregation and a DNA immobilized chip is set in each container in the aggregation. Thus, it is utilized as a cassette type DNA library.

By utilizing these cassette type DNA library, the above described comparisons ① and ② can be systematically operated so that change of gene can be effectively searched.

After sufficiently cleaning DNA immobilized chips according to the present invention in TE buffer solution and ethanol solution of 70∼75%, the chips are dampened in ethanol of 100% and preserved in a frozen condition, the libraries can be utilized for half eternal corresponding to demand of comparison data.

### (Amplifying DNA with DNA-immobilized chips)

A reaction container is formed by utilizing a plurality of the above cDNA immobilized chips. The reaction container is contacted to heating/cooling means under control. After an inner surface of the reaction container is sufficiently cleaned with TE buffer solution, a primer with respect to amplifying target DNA is set and PCR reaction solution including four kinds of nucleotide and DNA polymerize is added. After the reaction container is momentary increased to thermal metamorphism temperature of 95°C so as to separate a double chain DNA to a single chain DNA. The container is hold at a temperature at 95°C for about 1.5 minute. Then, the container is momentary cooled to annealing temperature for connecting a single chain DNA and DNA primer of 45°C. The container is held at 45°C for about 1 minute. Then, the container is increased to DNA amplifying temperature for extending a DNA chain by heat resistant DNA polymerize of 74°C. The container is held at the temperature for about two minute. The above thermal cycle is repeated 30times to operate PCR. Total time period of PCR is sum of holding period of about 135 minute, since it is not necessary time for increasing/decreasing temperature at all.

### (EXAMPLE 8)

Vapor phase synthetic diamond obtained in the embodiment 1 is cut by laser beam so as to provide a test piece of 10mmx10mm.

After a surface of the test piece is treated with hydrogen plasma energized by micro wave plasma, as similar as the embodiment 2, the surface of the diamond is chloridized.

After air is substituted by argon gas, the test piece is picked up and boiled in hydroxyl potassium solution of 10wt% for 15minute for hydroxylation. On the other hand, 95% ethanol 5% solution of 100cc is PH controlled to PH5 by added acetic acid. While 3-gricydxypropyrutrimethoxyl of 2cc is added and stirred, the hydroxyl diamond is immersed in the solution. After picking up the hydroxyl diamond, the diamond is lightly cleaned with ethanol so that a epoxy radical is introduced to a surface of the diamond by treating at 110°C for 5minute.

### (EXAMPLE 9)

With respect to the epoxy radical, 5' terminal amination oligo nucleic acid (primer) is immobilized. After annealing mRNA, cDNA replica is produced by RT enzyme.

### (EXAMPLE 10)

A vapor phase synthetic diamond obtained in the embodimetn 1 is divided to a test piece of 10mm x 10mm by laser beam.

After a surface of the test piece is surface treated by hydrogen plasma energized by micro wave plasma, as similar as the embodiment 2, a surface of the diamond is chloridized.

After air is substitute by argon gas, the test piece is picked up and boiled in hydroxyl potassium solution of 10wt% for 15minute for hydroxylation of the surface of the diamond.

On the other hand, 3-aminopropyletrimethoxylcilane of 2cc is stirred and added to 95% ethanol 5% solution of 100cc is added. After picking up the hydroxyl diamond, the diamond is lightly cleaned with ethanol so that an amino radical is introduced to a surface of the diamond by treating at 110°C for 5minute.

### (EXAMPLE 11)

After oligo nucleic acid modified carboxyl radical at a 5' terminal is immobilized on a surface of a diamond introduced with amino radical obtained in the embodiment 10 with a peptide linkage, cDNA is immobilized on the diamond by RT enzyme while mRNA is utilized as a casting mold.

### (EXAMPLE 12)

Aluminum nitride circular board of which a diameter is 64mm and a thickness is 0.1mm, surface roughness Ra is 0.3mm is vapor sythtized by ark ion plating method. A test piece of 3mm x 5mm is cut off from the circular board by laser beam. As the result of thermal conductive ratio, a value of 1.70W/cm·K can be obtained. The test piece is set in a separable flask and air in the flask is substituted by argon gas. While chloride gas is flown into the flask at 1SCCM, a surface of the test piece is chloridized by irradiating ultraviolet of which main wavelength is 3600 Å for 60 minute. The air is substituted by argon gas, the test piece is picked up and boiled in sodium hydroxide of 10wt% for 15 minute, further cleaned with water and dried. As the result, a substrate having hydroxyl radical at a terminal can be obtained. Then, as similar as the embodiment 1, DNA immobilizing and amplifying utilized by PCR method are operated by the substrate.

### (EXAMPLE 13)

A tungsten circular plate of which diameter is 64mm, a thickness is 0.5mm as average surface roughness is obtained by a power-sintering method. A test piece of 3mm x 5mm is divided from the circular plate. As a measured result of thermal conductive ratio, a value of 1.67W/cm·K can be obtained. A surface of the test piece is chloridized by oxygen plasma energized by microwave. After air is substituted by argon gas, the test piece is picked up and boiled in sodium hydroxide solution for 15 minute. Then, the test piece is cleaned with water and dried so that a substrate having hydroxyl radical at terminal can be obtained. After that, as similar as the embodiment 1, DNA immobilization and amplification by PCR method are operated by utilizing the substrate.

### (EXAMPLE 14)

An alumina circular plate is produced by a power sintering method. A test piece of 3mm x 5mm is divided from the circular plate by laser beam. As a result of thermal conductive ratio, a vale of 0.3W/cm·K can be obtained. The test piece is set in a separable flask and air in the flask is substituted by argon gas. While chloride gas is flown into at 1SCCM, a surface of the test piece is chloridized by irradiating ultra violet beam of which main wavelength is 3600Å for 60 minute. Air in the flask is again substituted by argon gas, N,N-dimethyleholmaldehyde solution of 100ml of sebacic soda of 1wt% is add. A condenser is set in the separable flask and refluxed for 2 hours. Then, the test piece is picked up, cleaned with acetic acid solution of 1wt%. The test piece is cleaned with acetone and dried so that it can be obtained a substrate with cebacic acid connected through a ester linkage and carboxyl radical at terminal. After that, as similar as the embodiment 1, DNA immobilization and amplification by PCR method by utilized the substrate.

In addition to contact the container with heating/cooling means such as a thermister as described above, substrates and DNA chips according to the present invention may be inserted into reaction solution is an Eppen type tube as similar as DNA amplification in a conventional PCR method.

In such a case, comparing to the above embodiments, a PCR amplification method can not be finished for a short time.

However, such a period is shorter than that of a conventional method for inserting DNA into reaction solution.

### POSSIBILITY OF USE IN INDUSTRY

In the present invention, substrates having excellent thermal conductive ratio is utilized so that DNA amplification reaction can be finished for an extremely short period compared to that of the PCR method.

By directly contacting the substrates with heating/cooling means, accuracy of thermal control of the above PCR reaction can be improved so that DNA expect target DNA amplification can not be reproduced. It is an advantage.

In substrate according to the present invention, DNA is directly immobilized on a solid state substrate with excellent thermal conductive ratio so that following characteristic of heat cycle caused by the PCR method and so on can be improved by directing contacting the the substrate with heating/cooling means.

In substrates according to the present invention, a surface is chemically modified with hydroxyl radical, carboxyl radical, epoxy radical and amino radical. Therefore, DNA immobilization becomes stable and it is the most suitable for chips for reproducing DNA by DNA amplification reaction by utilizing the PCR method and so on.

In substrates according to the present invention, chemical modification is reproduced by hydrolysis in the case that a surface is contaminated. Therefore, expensive DNA chip can be saved.

## Claims

1. Solid state substrate for DNA immobilizing with excellent thermal conductive characteristic for amplifying immobilized DNA.

2. Substrate as claimed in claim 1, wherein said substrate is diamond.

3. Substrate as claimed in claim 1 or 2, wherein said substrate is chemically modified.

4. Substrate as claimed in one of claims 1 through 3, wherein said substrate has a polar group at terminal.

5. Substrate as claimed in claim 4, wherein said polar radical is hydroxyl radical, carboxyl group, epoxy radical or amino radical.

6. Substrate as claimed in claim 5, wherein said carboxyl radical is connected on a surface of said substrate through ester linkage.

7. Substrate as claimed in claim 5, wherein said carboxyl radical is connected on a surface of said substrate through amide linkage.

8. Substrate as claimed in claim 5, wherein said carboxyl radical is introduced to a surface of said substrate with cylane coupling agent, titanium coupling agent or aluminum coupling agent.

9. Substrate as claimed in claim 5, wherein said epoxy radical is introduced to a surface of said substrate with cylane coupling agent, titanium coupling agent or aluminum coupling agent.

10. Substrate as claimed in claim 5, wherein said amino radical is introduced to a surface of said substrate with cylane coupling agent, titanium coupling agent or aluminum coupling agent.

11. Chip for immobilizing DNA as claimed in claim one of claims 1 to 10, wherein DNA is immobilized on said substrate.

12. Method for amplifying DNA for substrate as claimed in claims 1 through 10 and chip as claimed in claim 11.
